# EUROPÄISCHE PATENTSCHRIFT

(11) **EP 2 872 484 B1**
(45) Veröffentlichungstag und Bekanntmachung des Hinweises auf die Patenterteilung: **08.08.2018**
(21) Anmeldenummer: 13734699.5
(22) Anmeldetag: 25.06.2013
(51) Int. Cl.: C07C 319/18

(54) **THIOVERETHERUNG VON MERCAPTANEN IN C4-KOHLENWASSERSTOFFGEMISCHEN**
THIOETHERIFICATION OF MERCAPTANES IN C4 HYDROCARBON MIXTURES
THIO-ÉTHÉRIFICATION DE MERCAPTANS DANS DES MÉLANGES D'HYDROCARBURES EN C4

(30) Priorität: 13.07.2012 DE 102012212317
(43) Veröffentlichungstag der Anmeldung: 20.05.2015
(73) Patentinhaber: Evonik Degussa GmbH, 45128 Essen (DE)
(72) Erfinder: PEITZ, Stephan, 45739 Oer-Erkenschwick (DE); WINTERBERG, Markus, 45731 Waltrop (DE); MASCHMEYER, Dietrich, 45657 Recklinghausen (DE); GEILEN, Frank, 45721 Haltern am See (DE); BUKOHL, Reiner, 45770 Marl (DE); SCHALLENBERG, Jörg, 46286 Dorsten (DE); RIX, Armin, 45770 Marl (DE); WOLFF, Andreas, 45657 Recklinghausen (DE); LEIPOLD, Matthias, 45721 Haltern am See (DE)
(86) Internationale Anmeldenummer: PCT/EP2013/063300
(87) Internationale Veröffentlichungsnummer: WO 2014/009148

(56) Entgegenhaltungen:
- WO-A1-03/062178
- WO-A2-03/104168
- DE-C- 740 247
- DE-C- 864 866
- US-A- 5 851 383

## Beschreibung

Die Erfindung betrifft ein Verfahren zur Thioveretherung von Mercaptanen mit mehrfach ungesättigten Kohlenwasserstoffen, durchgeführt in einem Reaktor unter Zusatz von Wasserstoff, unter Einsatz eines heterogenen Katalysators und in Gegenwart von 1-Buten. Bekannt ist ein solches Verfahren aus US5851383.

C₄-Kohlenwasserstoffe sind Verbindungen, die ausschließlich aus Kohlenstoff und Wasserstoff bestehen, wobei die Anzahl der Kohlenstoffatome je Molekül vier beträgt. Wichtige Vertreter der C₄-Kohlenwasserstoffe sind die Alkene und Alkane mit vier Kohlenstoffatomen.

Gemische aus C₄-Kohlenwasserstoffen sind Rohstoffe der Petro-Folgechemie. Sie stammen entweder aus Streamcrackern (so genanntes "Crack C4") oder aus fluidkatalytischen Crackern (so genanntes "FCC C4"). Es werden auch Gemische von C₄-Gemischen unterschiedlicher Herkunft gehandelt, so genannter "C₄-Schnitt". Zum Zwecke der Verwertung der einzelnen Komponenten sind die C₄-Gemische möglichst sortenrein in ihre Bestandteile zu zerlegen.

Mercaptane sind Verbindungen der Klasse R-SH, wobei R für einen Alkyl-Rest steht und S für Schwefel und H für Wasserstoff. Mercaptane werden auch als Thiole bezeichnet. Wichtige Vertreter der Mercaptane sind Methylmercaptan und Ethylmercaptan, auch Methanthiol bzw. Ethanthiol genannt. Mercaptane treten mit bis zu 1000 ppm als unerwünschte Begleitstoffe in C₄-Kohlenwasserstoffgemischen auf.

Technische C₄- Kohlenwasserstoffgemische aus katalytischen Crackern (FCC C4) oder Steamcrackern (Crack C4) enthalten üblicherweise neben gesättigten und einfach ungesättigten Verbindungen auch mehrfach ungesättigte Verbindungen. Bevor einzelne Verbindungen aus diesen Gemischen isoliert werden können, ist es häufig notwendig, andere Verbindungen möglichst vollständig zu entfernen. Dies kann durch physikalische Methoden, wie z. B. Destillation, Extraktivdestillation oder Extraktion, aber auch durch eine selektive chemische Umsetzung der zu entfernenden Komponenten erfolgen. Besonderes Augenmerk muss dabei auf der möglichst vollständigen Entfernung von den im C₄-Kohlenwasserstoffgemisch enthaltenden Verunreinigungen, wie Sauerstoff-, Stickstoff- und Schwefelhaltigen Komponenten liegen, da diese als Katalysatorgifte negative Auswirkungen auf die einzelnen Prozessschritte haben können. Während diese Verunreinigung typischerweise in Crack C4 nur in Spuren vorhanden sind, können diese in FCC C4-Strömen auch in höheren Konzentrationen vorhanden sein.

C₄-Kohlenwasserstoffgemische aus Steamcrackern oder Fluidized Catalytic Crackern weisen typischerweise die in Tabelle 1 aufgeführten Hauptkomponenten auf. (Verunreinigungen nicht dargestellt)

**Tabelle 1: Typische Zusammensetzungen von Crack C4 und FCC C4**

| **Komponente** | **Crack C4** [Massen-%] | **FCC C4** [Massen-%] |
|---|---|---|
| Isobutan | 1 - 3 | 20 - 40 |
| n-Butan | 6 - 11 | 5 - 15 |
| 1-Buten | 14 - 20 | 10 - 20 |
| 2-Butene | 4 - 8 | 20 - 35 |
| Isobuten | 20 - 28 | 10 - 20 |
| 1,3-Butadien | 40 - 45 | kleiner 1 |

Die Zusammensetzung der Rohstoffe kann je nach Herkunft des Materials stark schwanken. Zu den aufgeführten C₄-Komponenten gesellen sich noch Kohlenwasserstoffe mit weniger oder mehr Kohlenstoffatomen, sowie Verunreinigungen wie Mercaptane, Sulfide, Disulfide, Stickstoff- und sauerstoffhaltige Verbindungen in geringen Mengen.

Die Aufarbeitung von FCC C4 kann in einer Variante so erfolgen, dass zunächst die Konzentration des Isobutans mittels eines destillativen Schrittes in einer Destillation auf einen Wert von kleiner 5 Massen%, besonders bevorzugt kleiner 3 Massen-% gesenkt wird. Gleichzeitig werden die im Gemisch vorhandenen Leichtsieder (z. B. C₃-Kohlenwasserstoffe, leichte Sauerstoff, Stickstoff- und Schwefelhaltige Verbindungen) entfernt bzw. minimiert. Im darauf folgenden Schritt werden in einer Kolonne alle Hochsieder (z.B. C₅-Kohlenwasserstoffe, schwere Sauerstoff-, Stickstoff- und Schwefelhaltige Verbindungen) über den Sumpf entfernt. Im nächsten Schritt wird Isobuten entfernt, z. B. indem es mit Methanol zu Methyl-tert.-butylether (MTBE) umgesetzt und dieser durch Destillation entfernt wird. Soll reines Isobuten gewonnen werden, kann der Methyl-tert.-butylether anschließend wieder zu Isobuten und Methanol gespalten werden.

Zur weiteren Aufarbeitung des C₄-Gemisches müssen die noch verbliebenen mehrfach ungesättigten Verbindungen mit Hilfe eines Selektivhydrierprozesses zu den entsprechenden einfach ungesättigten und gesättigten Verbindungen umgesetzt werden. Jetzt können 1-Buten und verbliebenes Isobutan in ausreichender Reinheit destillativ abgetrennt und die verbleibenden 2-Butene und das n-Butan weiter aufgearbeitet werden. Häufig werden die 2-Butene durch Dimerisierung zu Oktenen umgesetzt, die anschließend mittels Hydroformylierung zu PVC-Weichmacheralkoholen umgesetzt werden. Die gesättigten C₄-Kohlenwasserstoffe können beispielsweise als Treibmittel verwendet werden.

Wird im Selektivhydrierprozess vor der 1-Buten-Abtrennung die Konzentration der mehrfach ungesättigten Verbindung nicht auf einen Wert kleiner 10 ppm abgesenkt, so werden die Reinheitsanforderungen für 1-Buten, welches in Polymerisationen eingesetzt wird, nicht erreicht. Des Weiteren unterdrücken mehrfach ungesättigte Verbindungen die katalytische Aktivität der Katalysatoren für die Dimerisierung der 2-Butene.

Die Aufarbeitung von C₄-Strömen aus Steamcrackern oder Katalytischen Crackern wird prinzipiell beschrieben in K.-D. Wiese, F. Nierlich, DGMK-Tagungsbericht 2004-3, ISBN 3-936418-23-3.

Die Ansprüche an die Selektivitäten bei den Prozessen zur selektiven Hydrierung von mehrfach ungesättigten Kohlenwasserstoffen sind besonders hoch, da bei der Überhydrierung, d.h. der Hydrierung von einfach ungesättigten Verbindungen, sowie der Isomerisierung von endständigen Doppelbindungen zu innenständigen Doppelbindungen Wertprodukte vernichtet werden. Gleichzeitig müssen bei der Feinreinigung von Strömen, die bereits einen niedrigen Gehalt an mehrfach ungesättigten Verbindungen aufweisen, die Konzentrationen an mehrfach ungesättigten Verbindungen weiter auf Werte unter 10 ppmw abgesenkt werden.

Prozesse bzw. Katalysatoren zur Selektivhydrierung von 1,3-Butadien in hoher Konzentration (etwa 30 bis 50%) in C₄-Strömen werden in EP0523482, DE3119850, EP0992284 und EP0780155 beschrieben.

Bei katalytischen C₄-Strömen kann es nun vorkommen, dass neben den bereits in der Leichtsiederabtrennung destillativ entfernten leichten Schwefelhaltigen Komponenten, wie z. B. H₂S, COS oder MeSH, und den bereits in der C₅-Kolonne abgetrennten höhersiedenden Schwefel-Verbindungen, wie z. B. Dimethyldisulfid, weiterhin Mercaptan-artige Mittelsieder (z. B. Ethanthiol) enthalten sind. Diese sind nicht ohne Weiteres destillativ aus dem C₄-Strom zu entfernen. Die Anwesenheit von Mercaptanen ist aus mehreren Gründen bei der Aufarbeitung von C₄-Strömen nicht erwünscht bzw. stört diese:
a) Sind Mercaptane (z. B. Ethanthiol) im Zulauf zur Selektivhydrierung vorhanden, so hemmen diese die katalytische Umsetzung des 1,3-Butadiens. So können sich die verzweigten mehrfach ungesättigten Verbindungen im nachfolgenden Produkt (z. B. 1-Buten) befinden und dessen Reinheit gefährden.
b) Gelangen die mehrfach ungesättigten Verbindungen aufgrund der mangelnden Umsetzung in der Selektivhydrierung, verursacht durch die Mercaptangehalt, in den Zulauf zur Oligomerisierung der n-Butene, so desaktivieren sie den Oligomerisierungskatalysator.
c) Gelangen die Mercaptane in den Zulauf zur Oligomerisierung der n-Butene, so desaktivieren sie den Oligomerisierungskatalysator.
d) Es ist bekannt, dass sich durch Beschickung des Selektivhydrierkatalysators mit Schwefelhaltigen Komponenten hydroisomerisierungsaktive Katalysatoren bilden können. Durch die im Zulauf zur Selektivhydrierung vorhandenen Mercaptane kann ein solcher Hydroisomerisierungskatalysator gebildet werden, der zu einer unerwünschten Isomerisierung des 1-Butens zu 2-Butenen führt.

Die hier beschriebene Erfindung macht es sich zur Aufgabe, diese Probleme bei der Aufarbeitung von C₄-Strömen zu umgehen.

Als Stand der Technik kann man bei der Gegenwart von Mercaptanen einen Prozess zur Extraktion der Mercaptane mit wässriger Laugenlösung und anschließender oxidativer Umwandlung der Mercaptanen zu Disulfiden durchführen, um die Konzentration der Mercaptane auf Werte ca. 5 - 15 ppm abzusenken. Ein derartiges Verfahren wird für den industriellen Einsatz von der Fa. UOP LLC unter dem Namen MEROX® angeboten. (G. A. Dziabis, "UOP MEROX PROCESS" in Robert Meyers, Handbook of Petroleum Refining Processes, 3rd Edition, 2004 McGraw-Hill)

Der Nachteil des MEROX®-Prozesses besteht darin, dass neben hohem apparativen Aufwand auch große Mengen an Wasser- und Laugenströmen anfallen, deren Entsorgung aufwändig ist. Zudem ist eine vollständige Abtrennung höherer Mercaptane (z. B. Ethanthiol) nicht gegeben. Zusätzliche Maßnahmen zur Entfernung dieser Restanteile an Mercaptanen müssen bei weiterer chemischer Verwendung daher zwingend vorgenommen werden. Dies geschieht etwa durch adsorbtive Entfernung. Adsorbtive Verfahren zur Entschwefelung von C₄-Strömen sind in DE3914817C2 und DE19845857A1 beschrieben.

Alternativ sind Prozesse zur Thioveretherung der Mercaptane mit ungesättigten Kohlenwasserstoffen bei gleichzeitiger Hydrierung der mehrfach ungesättigten Kohlenwasserstoffe und gleichzeitiger Isomerisierung des 1-Butens zu 2-Butenen bekannt, der die Konzentration der Mercaptane auf Werte kleiner 1 ppm und die der mehrfach ungesättigten C₄-Kohlenwasserstoffe auf Werte kleiner 10 ppm absenkt, sowie die Konzentration des 1-Butens ins Gleichgewicht zu 2-Buten setzt.

US5851383 beschreibt solches ein Verfahren zur simultanen Thioveretherung von Mercaptanen zu höhersiedenden Thioethern, selektiven Hydrierung von mehrfach ungesättigten Olefinen in FCC-C₃-C₅-Strömen und Isomerisierung von leichten Monoolefinen. Der Prozess wird in einem Festbettreaktor in rein flüssiger Phase durchgeführt und als Katalysator dient Nickel auf Aluminiumoxid. Wasserstoff wird in zweifachem molarem Überschuss zum Diolefin zugegeben.

US5463134 beschreibt ein Verfahren zur simultanen säurekatalysierten Thioveretherung von Mercaptanen mit Butenen zu höhersiedenden Thioethern und Entfernung von Olefinen durch Oligomeriserung in Abwesenheit von Wasserstoff in einem Paraffin-reichen C₄-Strom. Der Prozess wird in einem Festbettreaktor in rein flüssiger Phase durchgeführt und als Katalysator dient ein saurer Ionenaustauscher.

WO 2003062178 beschreibt ein Verfahren zur Vorbereitung von C₄-Strömen für eine Alkylierung von Isobutan mit Butenen. Dabei wird unter anderem eine simultane Thioveretherung von Mercaptanen zu höhersiedenden Thioethern, eine selektive Hydrierung von mehrfach ungesättigten Olefinen und eine Isomerisierung von leichten Monoolefinen vorgenommen. Der Prozess wird in einem Festbettreaktor in rein flüssiger Phase durchgeführt und als Katalysator dient Nickel auf Aluminiumoxid. Wasserstoff wird in zehnfachem molarem Überschuss zum Diolefin zugegeben.

Der Nachteil der genannten Thioveretherungsverfahren besteht darin, dass neben der Umsetzung der Mercaptane und Diolefine auch eine Isomerisierung von 1-Olefinen, speziell 1-Buten, stattfindet. Dies führt zu einer starken Abnahme des 1-Olefingehaltes, so dass eine nachfolgende, gezielte Isolierung der 1-Olefine nicht mehr möglich ist. 1-Olefine sind aber gefragte Wertprodukte, die Gewinn bringend isoliert und genutzt werden können.

Parallel zu der unerwünschten Isomerisierung dazu findet bei
WO2003062178 und bei US5851383 durch den im molaren Überschuss eingesetztem Wasserstoff auch eine Hydrierung der Monoolefine statt. Dies führt zu einer signifikanten Verringerung des Gesamt-Monoolefingehalts im Produktstrom, der somit nicht mehr für Folgereaktionen (z. B. Oligomerisierung) zur Verfügung steht.

Lichte dieses Standes der Technik liegt der Erfindung die Aufgabe zu Grunde, ein Verfahren der eingangs genannten Gattung so weiterzubilden, dass die Wertschöpfung aus dem eingesetzten C₄-Rohstoffstrom gesteigert wird.

Gelöst wird diese Aufgabe dadurch, dass der Wasserstoff der Reaktion so zudosiert wird, dass das molare Verhältnis von Wasserstoff zu mehrfach ungesättigten Kohlenwasserstoffen maximal eins beträgt.

Gegenstand der Erfindung ist mithin Verfahren zur Thioveretherung von Mercaptanen mit mehrfach ungesättigten Kohlenwasserstoffen, durchgeführt in einem Reaktor unter Zusatz von Wasserstoff, unter Einsatz eines heterogenen Katalysators und in Gegenwart von 1-Buten, bei welchem das molare Verhältnis von Wasserstoff zu mehrfach ungesättigten Kohlenwasserstoffen maximal eins beträgt.

Der erfindungsgemäße Prozess ist in der Lage, Mercaptane unter vollständigem Umsatz in hochsiedende Thioether umzusetzen, gleichzeitig eine signifikante Isomerisierung von 1-Buten zu innenständigen Butenen nahezu vollständig zu unterdrücken, sowie eine Hydrierung der Butene vollständig zu verhindern.

Entgegen der Erwartung des Fachmanns wird im Rahmen der vorliegenden Erfindung gezeigt, dass Mercaptane in Gegenwart von 1,3-Butadien und Wasserstoff bis unter die Nachweisgrenze zu höhersiedenden Thioethern umgesetzt werden können, wobei die Isomerisierung von 1-Buten stark zurück gedrängt und die Hydrierung von Butenen komplett verhindert wird. Die Nachweisgrenze der Mercaptane liegt derzeit bei etwa 50 ppbw, also bei einem Gewichtsanteil von 50 *10⁻⁹.

Im Rahmen der vorliegenden Erfindung ist die einzuhaltende Wasserstoffmenge maximal äquimolar zu den im Kohlenwasserstoffgemisch enthaltenden mehrfach ungesättigten Kohlenwasserstoffen. Das molare Verhältnis von Wasserstoff zu den mehrfach ungesättigten Kohlenwasserstoffen liegt zwischen 0,01 und 0,8. Besonders bevorzugt liegt es zwischen 0,1 und 0,5.

Ein großer Vorteil dieses Verfahrens ist, das aufgrund des geringen Wasserstoffanteils im C₄-Strom enthaltendes 1-Buten kaum isomerisiert wird und weiterhin als Wertprodukt zur Verfügung steht. Darüber hinaus macht das Verfahren eine kostenintensive MEROX®-Wäsche verzichtbar. Nur durch die genau einzuhaltenden Grenzen für die zugeführte Wasserstoffmenge wird erreicht, dass Mercaptane in einem Verfahren auf Konzentrationswerte von unter 50 ppbw mit mehrfach ungesättigten C₄-Kohlenwasserstoffen zu hochsiedenden Thioethern verethert werden können, ohne dass dabei eine Hydrierung der ebenfalls im Zulauf enthaltenen einfach ungesättigten Butene sowie eine nennenswerte Isomerisierung des 1-Butens erfolgt.

Ein wesentliches Kennzeichen des Verfahrens ist es, dass ohne Wasserstoff kein Umsatz an Mercaptanen erfolgt.

Bei den mehrfach ungesättigten Kohlenwasserstoffen, welche mit den Mercaptanen thioverethert werden, handelt es sich vorzugsweise um 1,3-Butadien, und/oder um But-3-en-1-in und/oder um 1,2-Butadien. Diese Diene und Acetylene sind insbesondere in FCC-C4 nur in geringen Mengen vorhanden und müssen stromabwärts ohnehin vollständig hydriert werden und stehen damit nicht mehr als Wertprodukt zur Verfügung. Aus stark 1,3-Butadien-halten Crack C4-Strömen wird das 1,3 Butadien zuvor gesondert entfernt und verwertet. Die dabei im C₄-Strom verbleibenden Rest-Butadiene können dann für die Thioveretherung genutzt werden.

Ein besonderer Vorteil des Verfahrens ist, dass es neben dem hochreaktiven Mercaptan Methanthiol auch hinsichtlich der höheren Mercaptane (z.B. Ethanthiol) reaktiv ist. Bevorzugt werden also die im Strom enthaltende Mercaptane Methanthiol und/oder Ethanthiol mit mehrfach ungesättigten Kohlenwasserstoffen thioverethert.

Dem zu hydrierenden Kohlenwasserstoffgemisch kann optional zusätzlich Kohlenmonoxid zugegeben werden. Der Gehalt an Kohlenmonoxid im Zulauf beträgt in diesem Fall zwischen 0.05 und 20 ppm Kohlenmonoxid, bezogen auf die Masse des Kohlenwasserstoffgemisches. Bevorzugt wird zwischen 0.5 und 5 ppm Kohlenmonoxid zugegeben. Dosierungen oberhalb von 20 ppm verbessern die Ergebnisse nicht mehr. Das Kohlenmonoxid wird separat in den Reaktor eindosiert oder dem einlaufenden C₄-Strom mitgegeben.

Kohlenmonoxid wirkt als zusätzlicher Moderator, der eine Isomerisierung von 1-Buten zu 2-Butenen reduziert.

Als Katalysator zur Tioveretherung eignen sich heterogene Katalysatoren, die ein Metall der VIII. Gruppe des Periodensystems der Elemente enthalten.

Grundsätzlich ist die erfindungsgemäße Thioveretherung an keinen bestimmten Gruppe-VIII-Metall-Katalysator gebunden. Vorzugsweise liegt das Metall in geträgerter Form auf einem inerten Trägermaterial vor. Bei dem Trägermaterial handelt es sich beispielsweise um Aluminiumoxid, Kieselgel oder Aktivkohle. Bevorzugt wird Aluminiumoxid als Trägermaterial eingesetzt.

Ist der eingesetzte Katalysator ein Katalysator auf Basis von Palladium, so hat er eine Palladium-Konzentration, die zwischen 0,01 und 3%, bezogen auf die Masse des Trägers, liegt. Bevorzugt liegt sie zwischen 0,1 und 1%, ganz besonders bevorzugt zwischen 0,3 und 0,5%. Der Katalysator hat eine Innere Oberfläche (bestimmt durch Gasadsorption nach DIN ISO 9277) von 50 bis 400 m²/g, bevorzugt zwischen 100 und 300 m²/g, besonders bevorzugt zwischen 200 und 300 m²/g.

Als besonders vorteilhaft haben sich Schalenkatalysatoren zur Thioveretherung erwiesen, welche Aluminiumoxid als Träger und Palladium als katalytisch wirksames Metall umfassen.

Die Eintrittstemperatur des Reaktorzulaufs liegt vorzugsweise im Bereich von 0 bis 180°C, bevorzugt im Bereich von 60 bis 150°C, besonders bevorzugt im Bereich von 80 bis 130°C. Der Druck liegt vorzugsweise im Bereich von 0,2 bis 5 MPa, bevorzugt im Bereich von 0,6 bis 4 MPa, besonders bevorzugt im Bereich von 1 bis 3 MPa. In allen Fällen muss der Druck so gewählt werden, dass der Wasserstoff vollständig gelöst bleibt und im Reaktor keine Gasphase auftritt.

Die Thioveretherung wird vorzugsweise als Flüssigphasenverfahren betrieben. Dies bedeutet, dass sämtliche Komponenten am Katalysator in flüssiger Phase vorliegen oder flüssig in den Reaktor eingebracht werden. Insbesondere bedeutet das, dass der Wasserstoff und ggf. auch das Kohlenmonoxid in der flüssigen Phase vollständig gelöst sind.

Das Zugeben von Wasserstoff zu dem zu hydrierenden Gemisch aus Kohlenwasserstoffen erfolgt somit in fein verteilter Form und in solchen Mengen, bei denen stets eine homogene Flüssigphase vor Eintritt in den Hydrierreaktor vorhanden ist.

Die zu verethernden Kohlenwasserstoffgemische können bis zu 1000 wppm Mercaptane enthalten, also ein Gewichtsanteil von 10⁻³. Die Thioveretherung kann in einer oder mehreren Reaktionsstufen durchgeführt werden. Falls eine so große Menge an Mercaptanen im Zulauf enthalten ist, dass die notwendige Menge Wasserstoff nicht mehr im Zulauf löslich ist, so kann der Zulauf durch Kreislauffahrweise verdünnt werden. Alternativ kann der Wasserstoff in mehreren Teilmengen, verteilt über die Reaktorlänge bzw. über die einzelnen Reaktionsstufen, zugegeben werden.

Nach Vollumsatz der Mercaptane zu hochsiedenden Thioethern ist eine destillative Abtrennnung dieser Thioether möglich. Dadurch verringert sich der Thioethergehalt des verbleibenden C₄-Kohlenwasserstoffgemisches auf unter 50 ppbw. Zusammen mit einem möglichen dem Thioveretherungsreaktor vorgeschalteten Leichtsiederabtrennung und einer dem Thioveretherungsreaktor nachgeschalteten Schwersiederdestillation ist somit eine vollständige Entfernung aller Schwefelhaltigen Komponenten aus dem C₄-Kohlenwasserstoffgemisch möglich.

Die Konzentration an mehrfach ungesättigten Olefinen kann mittels GasChromatographie online gemessen und die Wasserstoffmenge exakt danach eingestellt werden. Dies gilt ebenfalls für die Schwefelverbindungen.

Das Verfahren wird bevorzugt auf Mercaptan-haltige Gemische aus C₄-Kohlenwasserstoffen angewendet, die aus katalytischen Crackern (FCC C4) oder aus Steamcrackern (Crack C4) stammen. Selbstverständlich kann auch C4-Schnitt verarbeitet werden.

Bevorzugt wird das als Zulauf verwendete C₄-Kohlenwasserstoffgemisch zuvor einer destillativen Abtrennung von Leichtsiedern, insbesondere von Isobutan unterzogen.

Alternativ wird das Verfahren vor der Abtrennung von Isobutan angewendet.

Nach der erfindungsgemäßen Thioveretherung folgt im Zuge der weiteren Aufarbeitung und Verwertung des C₄-Stroms mindestens einer dieser Verfahrensschritte:
- Destillative Abscheidung von Thioethern;
- Adsorbtive Entfernung von Schwefelkomponenten;
- Selektivhydrierung von 1,3-Butadien zu 1-Buten und/oder zu 2-Buten;
- Destillative Abtrennung von 1-Buten;
- Oligomerisierung von 2-Butenen zu Olefinen mit mehr als 4 Kohlenstoffatomen;
- Destillative Abtrennung von n-Butan und/oder von Isobutan;
- Veretherung von Isobuten mit Methanol zu Methyl-tert.-Butylether (MTBE) und Abtrennung des entstandenen MTBE.

Es können auch mehrere der aufgeführten Verarbeitungsschritte hinter der Thioveretherung angeordnet werden. Die Reihenfolge kann - je nach Zusammensetzung des verarbeiteten Stromes - unterschiedlich gewählt werden.

Bei der Verarbeitung von FCC C4 ist diese Reihenfolge besonders bevorzugt:
1. Destillative Abscheidung von Thioethern;
2. Adsorbtive Entfernung von Schwefelkomponenten;
3. Veretherung von Isobuten mit Methanol zu MTBE und Abtrennung des entstandenen MTBE
4. Selektivhydrierung von 1,3-Butadien zu 1-Buten und/oder 2-Buten;
5. Destillative Abtrennung von 1-Buten;
6. Oligomerisierung von 2-Butenen zu Olefinen mit mehr als 4 Kohlenstoffatomen.

Die destillative Abscheidung der Thioether geschieht fachüblicherweise in einer Destillationskolonne. Bevorzugt werden zugleich den C₄-Strom begleitende Schwersieder wie C₅-Kohlenwasserstoffe zusammen mit den Thioethern abgeschieden. Der entschwefelte C₄-Strom wird über Kopf der Destillationskolonne abgezogen.

Eine in diesem Verfahrensschritt bevorzugt eingesetzte Destillationskolonne weist vorzugsweise von 40 bis 150 theoretische Trennstufen, bevorzugt von 40 bis 100 und besonders bevorzugt von 50 bis 80 theoretische Trennstufen auf. Das Rücklaufverhältnis beträgt, in Abhängigkeit von der realisierten Stufenzahl, der Zusammensetzung des Kolonnenzulaufes und der erforderlichen Reinheiten von Destillat und Sumpfprodukt, vorzugsweise zwischen 0,5 und 5, besonders bevorzugt zwischen 1 und 2,5. Das Rücklaufverhältnis ist hierbei definiert als Massenstrom des Rücklaufs geteilt durch den Massenstrom des Destillats. Die Kolonne wird vorzugsweise mit einem Betriebsdruck von 0,1 bis 2,0 MPa (absolut), bevorzugt von 0,5 bis 1,2 MPa (absolut) betrieben. Zur Beheizung der Kolonne kann z. B. Dampf eingesetzt werden. Die Kondensation kann, je nach gewähltem Betriebsdruck, gegen Kühlsole, Kühlwasser oder Luft erfolgen. Der Kopfbrüden der Kolonne kann aber auch mit anderen Kolonnen im Verfahren, z.B. mit der Kolonne zur Abtrennung des Isobutans, wärmeintegriert werden. In diesem Fall dient der Kondensator der Kolonne gleichzeitig als Verdampfer der Leichtsiederkolonne. Das Sumpfprodukt kann thermisch verwertet werden oder als Einsatzstoff anderer Prozesse, beispielsweise in einer Synthesegasanlage, genutzt werden.

Grundsätzlich lassen sich mit der erfindungsgemäßen Thioveretherung und der darauffolgenden destillativen Abscheidung der entstandenen Thioether sämtliche katalysatorschädlichen Mercaptane entfernen. Da die Auswirkungen von kleinsten Restmengen an Schwefelverbindungen im darauffolgenden Prozess aber schwerwiegende Schäden verursachen kann, sollte die Schwefelabscheidung redundant ausgelegt werden. Hierzu wird vorzugsweise ein Adsorber-Bett vorgesehen, durch welchen der Kopfstrom der destillativen Thioether-Abscheidung hindurchgeleitet wird und dabei kleinste Restmengen an schwefelhaltigen Verbindungen adsorbiert. In der Regel wird der Adsorber kaum belegt. Im Falle einer Betriebsstörung in der Thioveretherung oder der darauffolgenden Destillation hält der Adsorber auch eine große Schwefelverbindungs-Fracht von darauffolgenden katalytischen Verarbeitungsschritten fern. Geeignete Adsorbentien zur Entschwefelung von C₄-Strömen sind in DE3914817C2 oder in DE3825169A1 und DE19845857A1 beschrieben.

Die adsorbtive Entschwefelung wird vorzugsweise bei einem Druck von 0,1 bis 5 MPa und einer Temperatur von 20 bis 160°C in der Flüssigphase durchgeführt. Typische Spurenkomponenten, die über die Reinigung mit dem Adsorber entfernt werden, sind z. B. Schwefel-, Stickstoff-, Sauerstoff- und/oder Halogenverbindungen.

Aus dem so gewonnen C₄-Gemisch wird im erfindungsgemäßen Verfahrens bevorzugt das enthaltene Isobuten mit Methanol an saueren Ionentauschern unter Erhalt einer MTBE-Reaktionsmischung umgesetzt und das MTBE abgetrennt. Prinzipiell können hierzu alle bekannten Verfahren zur MTBE-Synthese eingesetzt werden, beispielsweise kann die MTBE-Synthese analog zur Beschreibung in DE10102082A1 erfolgen.

Verbleibendes Butadien, welches nicht mit den Mercaptanen zu Thioethern umgesetzt wurde, wird bevorzugt in einem Hydrierschritt selektiv zu Butenen umgesetzt. Da in C₄-Strömen 1,3-Butadien das häufigste Butadien ist, wird es - nach Möglichkeit ohne Hydrierung der übrigen Olefine - zu 1-Buten und/oder 2-Buten hydriert.

Besonders bevorzugt erfolgt die Selektivhydrierung des 1,3-Butadiens bei etwa 40°C in der Flüssigphase, bei einem molaren Verhältnis von 1,3-Butadien zu Wasserstoff von 1.1 und unter Zugabe von 1 ppm Kohlenmonoxid an einem Palladium-Katalysator. Da sich diese Reaktionsbedingungen deutlich von denen der Thioveretherung unterscheiden, können beide Schritte nicht in demselben Reaktor erfolgen. Ein geeignetes Verfahren zur Selektivhydrierung von 1,3-Butadien zu 1-Buten und/oder 2-Buten ist in DE102010030990A1 offenbart. Auch DE3143647A1 zeigt ein geeignetes Verfahren.

Die Hydrierung erfolgt in flüssiger Phase an einem Palladium-haltigen Festbettkatalysator mit Wasserstoff unter Zusatz von Kohlenmonoxid als Moderator. Wasserstoff und Kohlenmonoxid sind dabei im Kohlenwasserstoffgemisch vollständig gelöst. Als Wasserstoffmenge wird mindestens die zugegeben, die stöchiometrisch für die Hydrierung der mehrfach ungesättigten Verbindungen zu den Monoenen notwendig ist. Sie lässt sich aus der Zusammensetzung des zu hydrierenden C₄-Stromes berechnen.

Die auf die Masse des zu hydrierenden C₄-Stromes zu beziehende CO-Menge beträgt mindestens 0,05 ppm. Mengen von über 20 ppm führen standardmäßig zu keiner weitern wesentlichen Verbesserung der Hydrierergebnisse, so dass Mengen von 0,05 bis 10 ppm bevorzugt sind. Die im jeweiligen Prozess optimal zu dosierende Menge an CO kann leicht experimentell, wie in DE3143647A1 beschrieben, ermittelt werden.

Der Katalysator für die Selektivhydrierung weist 0,1 bis 2 Massen-% Palladium auf einem Träger auf. Zu solchen Trägern gehören beispielsweise Aluminiumoxid, Silicagel, Alumosilikat und Aktivkohle. Pro Liter eingesetztem Katalysator wird vorzugsweise eine Kohlenwasserstoffmenge im Bereich von 5 bis 300 Litern durchgesetzt.

Die Temperatur, bei der die Hydrierung durchgeführt wird, beträgt von 0 bis 75 °C. Ganz besonders bevorzugt sind Temperaturen um 40°C.

Der Verfahrensdruck muss hinreichend groß sein, um die Flüssigphase bei der gewählten Temperatur aufrecht zu erhalten und um eine ausreichende Menge an Wasserstoff und Kohlenmonoxid in Lösung zu bringen. Der Reaktionsdruck beträgt kleiner 20 MPa, vorzugsweise kleiner 6 MPa, bevorzugt kleiner 2 MPa. Ein typischer Reaktionsdruck beträgt 1,5 MPa.

Bevorzugt wird die Hydrierung mehrstufig, besonders bevorzugt zweistufig, durchgeführt. Die Einspeisung von Wasserstoff erfolgt dabei vor jedem der Reaktoren, die von Kohlenmonoxid vorzugsweise in den ersten der Reaktoren. Die Reaktoren können unter Rückführung von Produkt betrieben werden.

Im Anschluss an die Selektivhydrierung bietet es sich an, das Wertprodukt 1-Buten abzutrennen. Dies kann durch Destillation in einer oder mehreren Destillationskolonnen erfolgen. In einer bevorzugten Ausführungsform erfolgt die Abtrennung des 1-Butens in zwei Destillationskolonnen. In der ersten Destillationskolonne wird zunächst eine Isobutan- und 1-Buten-reiche Fraktion aus dem C4-Gemisch als Kopfprodukt abgetrennt, in einer weiteren Destillationskolonne wird dann der Isobutan- und 1-Buten-reiche Strom aufgetrennt. In dieser Kolonnen wird sehr reines 1-Buten als Sumpfprodukt erhalten. Als Kopfprodukt wird eine Isobutan-reiche Fraktion erhalten, die zudem gegebenenfalls Leichtsieder (beispielsweise C₃-Kohlenwasserstoffe) enthält.

Mit dem in diesem Aufarbeitungsschritt hergestelltes, reines 1-Buten enthält vorzugsweise weniger als 5000 Massen-ppm, bevorzugt weniger als 2000 Massen-ppm und besonders bevorzugt weniger als 1500 Massen-ppm Isobuten und ist ein gefragtes Zwischenprodukt. Es kann beispielsweise als Comonomer bei der Herstellung von Polyethylen (LLDPE oder HDPE) sowie von Ethylen-Propylen-Mischpolymeren eingesetzt werden. Es findet weiterhin Einsatz als Alkylierungsmittel und ist Ausgangsstoff für die Herstellung von Butan-2-ol, Butenoxid, Valeraldehyd.

Neben dem 1-Buten fallen, je nach Ausgangszusammensetzung der C₄ Kohlenwasserstoffe, bei der destillativen Aufarbeitung des Stroms Isobutan-reiche Fraktionen an. Diese können weiter, vorzugsweise zu reinem Isobutan, aufgereinigt werden. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine. Eine Aufreinigung zu reinem Isobutan kann beispielsweise durch vollständige Hydrierung der noch enthaltenen Alkene zu Alkanen und anschließende Destillation erfolgen.

Weitere Hinweise zur Durchführung der 1-Buten-Abscheidung finden sich in DE102005062700A1 und DE102005062699A1.

Besonders anfällig für die im erfindungsgemäßen Verfahren entfernten Katalysatorgifte sind Oligomerisierungen. Es bietet sich daher an, den erfindungsgemäß von Mercaptanen befreiten C₄-Strom nachfolgend einer Oligomerisierung zu unterwerfen, im Zuge derer 2-Butene und ggf. auch verbliebendes 1-Buten zu Olefinen mit mehr als 4 Kohlenstoffatomen oligomerisiert werden.

In diesem Verfahrensschritt werden die Butene werden unter Erhalt von Oligomeren an einem heterogenen, Nickel, Silizium und Aluminium aufweisenden Katalysator, oligomerisiert. Das diesem Verfahrensschritt zu Grunde liegende Verfahren hat Eingang in die Literatur als OCTOL®-Prozess gefunden, der in Hydrocarbon Process., Int. Ed. (1986) 65 (2. Sect.1), Seiten 31 bis 33 sowie in den Schriften DE3914817, EP1029839 und DE102004018753 beschrieben wird.

Die Oligomerisierung erfolgt in Gegenwart von heterogenen Nickel-haltigen Trägerkatalysatoren. Als Trägermaterialien können die Katalysatoren beispielsweise Siliciumdioxid und Aluminiumoxid, Alumosilikate oder Zeolithe aufweisen. Derartige Katalysatoren sind in der Fachliteratur bekannt und beispielsweise in DE4339713A1 oder WO0137989 beschrieben.

Die Oligomerisierung wird bei (Reaktions-)Temperaturen von 0 bis 200 °C, bevorzugt 50 bis 130 °C, und Drücken von 0,1 bis 70 MPa, bevorzugt von 0,1 bis 10 MPa und besonders bevorzugt von 0,5 bis 3 MPa, durchgeführt.

Durch die Oligomerisierung der Butene werden als Oligomeren insbesondere Olefine mit acht, zwölf, sechzehn, zwanzig oder mehr Kohlenstoffatomen erhalten. Diese Olefine können z. B. zur Herstellung von Weichmacheralkoholen (C₉- oder C₁₃-Alkohole) oder von Alkoholen (C₁₃-, C₁₇- oder C₂₁-Alkohole) zur Herstellung von Waschmittelrohstoffen verwendet werden. Bevorzugt werden sie vor der weiteren Verarbeitung destillativ in eine oder mehrere Fraktionen aufgearbeitet, wobei bevorzugt die Trennung in eine Fraktion mit Dibutenen (hauptsächlich C₈-Olefine), eine Fraktion mit Tributen (C₁₂-Olefine) und eine Fraktion mit höheren Oligomeren (C₁₆₊-Olefine) erfolgt. Aus den Dibutenen sind durch Hydroformylierung, Hydrierung und Destillation Isononylalkohole erhältlich, die im großen Maße als Weichmacheralkohole Verwendung finden. Aus den Tributenen können durch analoge Umsetzungen Isotridecylalkohole erhalten werden. Aus der C₁₆₊-Fraktion sind durch Hydrierung zu den Parafinen Mischungen hochreiner Parafine zugänglich.

In einer besonders bevorzugten Ausführungsform der Erfindung folgt unmittelbar oder mittelbar nach der Thioveretherung eine kombinierte destillative Abtrennung von Thioethern und von 1-Buten. Insbesondere findet diese kombinierte Thioether- und 1-Buten-Abtrennung hinter einer Selektivhydrierung von Butadien statt. Die kombinierte destillative Abtrennung der Thioethern und von 1-Buten erfolgt dabei bevorzugt in einer Seitenabzugskolonne, von deren Kopf 1-Buten und ggf. Isobutan abgetrennt wird, währenddessen am Sumpf der Seitenabzugskolonne die Thioether anfallen. Vom Seitenabzug wird Raffinat III, ein von Butadien, von Isobuten und von 1-Buten weitgehend befreites C₄-Kohlenwasserstoffgemisch abgezogen und beispielsweise einer Oligomerisierung zugeführt. Der Vorteil einer kombinierten Abtrennung der Thioether und von 1-Buten in einer Seitenabzugskolonne besteht darin, dass eine Seitenabzugskolonne geringere Investitionskosten verursacht als zwei einzelne Kolonnen.

Sofern hinter der Thioveretherung eine MTBE-Synthese vorgesehen ist, bietet es sich an, den dabei aus Isobuten und Methanol gebildeten zu Methyl-tert.-Butylether (MTBE) gemeinsam mit den Thioethern als ein Ethergemisch destillativ abzutrennen. Diese bevorzugte Ausführungsform kombiniert die destillative MTBE-Abtrennung und die Abscheidung der Thioether in einem Schritt.

Allerdings erfolgt die Abtrennung der beiden Ether nicht getrennt in einer Seitenabzugskolonne, sondern in einer herkömmlichen Destillationskolonne, an deren Sumpf ein Ethergemisch anfällt, das im Wesentlichen aus MTBE und Thioethern besteht. Da die Thioether in geringem Umfang gebildet werden als MTBE, stellt das Ethergemisch genauer gesagt ein mit Thioether verunreinigtes MTBE dar.

Die dabei zu erwartende Verunreinigung des MTBEs mit Thioethern ist so gering, dass das Ethergemisch wie ein technisches MTBE den Kraftstoffpool bereichern kann. Eine gesonderte Auftrennung des Ethergemisches in Thioether und reines MTBE ist deswegen nicht erforderlich. Aus diesem Grund entfällt die gesonderte Abscheidung der Thioether, weil sie mit der ohnehin notwendigen MTBE-Abtrennung erfolgen kann: Die Investitions- und Betriebskosten der Anlage werden dadurch gesenkt.

Anhand der Figuren werden nun einige bevorzugte Ausführungsformen der vorliegenden Erfindung näher erläutert. Es zeigen:
- Figur 1:: Blockschema einer ersten Ausführungsform;
- Figur 2:: Blockschema einer zweiten Ausführungsform;
- Figur 3:: Blockschema einer dritten Ausführungsform;
- Figur 4:: Blockschema einer vierten Ausführungsform;
- Figur 5:: Blockschema einer fünften Ausführungsform.

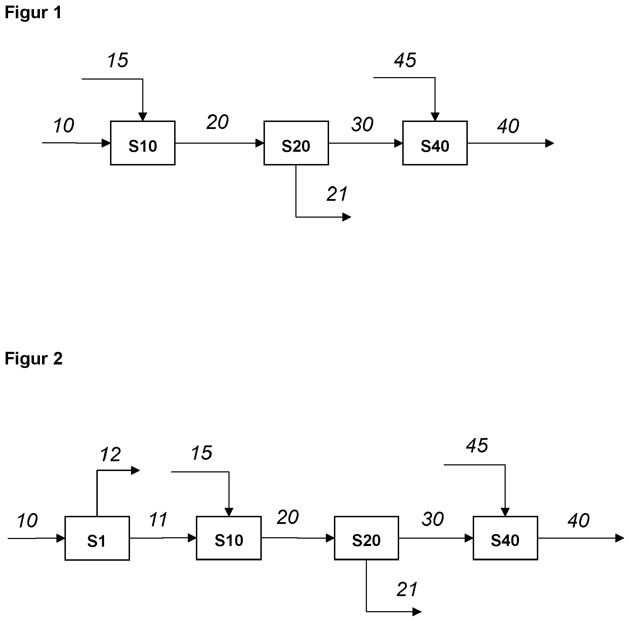

Ein Blockschema einer ersten bevorzugten Ausführungsform, mit der das erfindungsgemäße Verfahren durchgeführt werden kann, ist in Figur 1 dargestellt. Der mercaptanhaltige C₄-Kohlenwasserstoffstrom (10) wird Thioveretherungsschritt (S10) zugeführt. Optional wird diesem Schritt auch CO zugeführt. In den Thioveretherungsschritt wird auch Wasserstoff (15) zugeführt. In dem Thioveretherungsschritt werden die enthaltenen Mercaptane vollständig zu Thioethern umgesetzt, enthaltenes 1,3-Butadien wird nur teilweise umgesetzt.

Der mercaptanfreie Strom (20) wird einem Destillationschritt (S20) zugeführt, in dem die entstandenen Thioether vollständig mit weiteren höhersiedenen Komponenten, z.B. C₅-Kohlenwasserstoffe, als Sumpfstrom (21) abgetrennt werden. Sofern keine weiteren Schwersieder vorhanden sind, können die Thioether auch später zusammen mit anderen in der Folge gebildeten Schwersiedern, z.B. MTBE, abgetrennt werden. Im Falle einer direkten Schwersiederabtrennung (S20) wird der nun schwefelfreie Kopfstrom (30) wird einem Hydrierschritt (S40) zugeführt. In diesem Hydrierschritt wird noch enthaltenes 1,3-Butadien mit Wasserstoff (45) selektiv zu 1- und 2-Buten hydriert. Optional wird diesem Schritt auch CO zugeführt. Der nun schwefel- und 1,3-Butadien-freie C₄-Kohlenwasserstoffstrom (40) kann nun als Rohstoff in weiteren chemischen Produktionsverfahren eingesetzt werden.

Eine zweite bevorzugte Ausführungsform des Verfahrens ist in Figur 2 dargestellt. In dieser Verfahrensvariante werden aus dem C₄-Kohlenwasstoffstrom (10) in einem ersten Destillationssschritt (S1) Leichtsieder (12), vornehmlich Isobutan abgetrennt. Die weitere Aufarbeitung des weitestgehend leichtsiederfreien Stroms (11) erfolgt wie zuvor unter Figur 1 beschrieben.

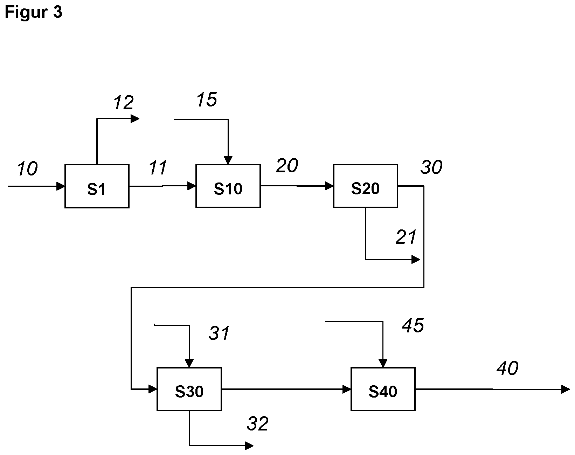

Eine dritte bevorzugte Ausführungsform des Verfahrens ist in Figur 3 dargestellt. In dieser Verfahrensvariante wird der Strom (30) nach der Hochsiederabtrennung (S20) einer Veretherungsstufe (S30) zugeführt. In dieser Stufe wird ein Alkohol (31), bevorzugt Methanol zugeführt und das enthaltene Isobuten zu einem Ether, bevorzugt Methyl-tert.-Butylether (MTBE) umgesetzt. Der Ether wird als Hochsieder (32) abgetrennt und der Isobutenfreie Strom (35) dem schon zuvor beschriebenen Hydrierschritt (S40) zugeführt.

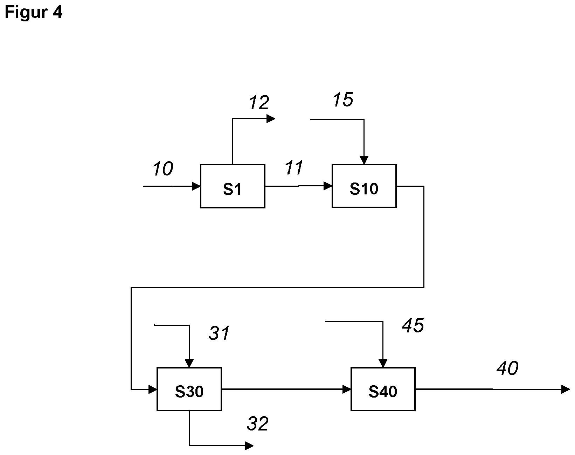

Eine vierte bevorzugte Ausführungsform des Verfahrens ist in Figur 4 dargestellt. Wie die in Figur 3 gezeigte Ausführungsform umfasst dieser Prozess eine hinter der Thioveretherung (S10) angeordnete MTBE-Synthese (S30). Allerdings werden die Thioether nicht in einer gesonderten Trennstufe entsprechend dem Destillationsschritt (S20) der dritten Ausführungsform ausgeschleust, sondern zusammen mit dem MTBE als hochsiedernes Ethergemisch (32) aus der Veretherungsstufe (S30).

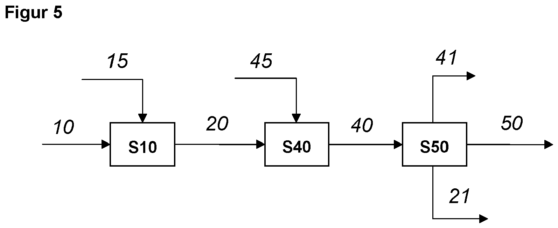

Eine fünfte bevorzugte Ausführungsform des Verfahrens ist in Figur 5 dargestellt. Der mercaptanhaltige C₄-Kohlenwasserstoffstrom (10) wird Thioveretherungsschritt (S10) zugeführt. Optional wird diesem Schritt auch CO zugeführt. In den Thioveretherungsschritt wird auch Wasserstoff (15) zugeführt. In dem Thioveretherungsschritt werden die enthaltenen Mercaptane vollständig zu Thioethern umgesetzt, enthaltenes 1,3-Butadien wird nur teilweise umgesetzt.

Der mercaptanfreie Strom (20) wird dann einem Hydrierschritt (S40) zugeführt. In diesem Hydrierschritt wird noch enthaltenes 1,3-Butadien mit Wasserstoff (45) selektiv zu 1- und 2-Buten hydriert. Optional wird diesem Schritt auch CO zugeführt. Der nun Mercaptan- und 1,3-Butadien-freie C₄-Kohlenwasserstoffstrom (40) wird dann in eine Seitenabzugskolonne (S50) eingespeist, in der eine kombinierte Abtrennung von 1-Buten und Isobutan (41) über Kopf erfolgt. Von Seitenabzug wird Raffinat III (50) abgezogen. Die Thioether 21 werden vom Sumpf der Seitenabzugskolonne (S50) abgezogen. In der Seitenabzugskolonne (50) erfolgt mithin eine kombinierte destillative Abtrennung von Thioethern und von 1-Buten.

Nachfolgend wird die vorliegende Erfindung anhand von Beispielen näher erläutert.

Alternative Ausführungsformen der vorliegenden Erfindung sind in analoger Weise erhältlich.

Die Thioveretherung wird in einem Festbettreaktor mit Heizmantel, durch den ein Wärmeträgeröl (Marlotherm SH der Sasol Olefins & Surfactants GmbH) fließt, durchgeführt. Als Katalysator werden 0,54 Liter eines Schalenkatalysators mit 0,5% Palladium auf γ-Aluminiumoxid in Strangform verwendet. Bei dem Katalysator handelt es sich um den bei der Evonik Industries AG erhältlichen NOBLYST® H1427-1.

Die spezifische innere Oberfläche des Katalysators beträgt ca. 250 m²/g und das Porenvolumen ca. 0,8 cm³/g. Die Dicke der Palladium-Schicht beträgt ca. 0,05 mm. Zur Herstellung des zu thioverethernden Gemisches aus C₄-Kohlenwasserstoffen werden Raffinat III, 1,3-Butadien und Ethanthiol gemischt. Edukt- und Produktgemisch werden gaschromatographisch analysiert.

### Beispiel 1 (gemäß Erfindung)

| Komponente | 1,3-Butadien | 1-Buten | 2-Butene | n-Butan | Ethanthiol |
|---|---|---|---|---|---|
| Zulauf [Gew.-%] | 0,513 | 27,710 | 44,987 | 26,292 | 0,00210 |
| Austrag [Gew.-%] | 0,345 | 27,552 | 45,325 | 26,284 | 0,00000 |

**Reaktionsbedingungen**

| T [°C] | P [bar] | Verhältnis n(H₂)/n(Dien) | Isomerisierung 1-Buten [%] |
|---|---|---|---|
| 91 | 24 | 0,30 | 0,57 |

### Beispiel 2 (Vergleichsbeispiel)

| Komponente | 1,3-Butadien | 1-Buten | 2-Butene | n-Butan | Ethanthiol |
|---|---|---|---|---|---|
| Zulauf [Gew.-%] | 0,485 | 26,220 | 45,182 | 27,652 | 0,00200 |
| Austrag [Gew.-%] | 0,0 | 14,454 | 56,943 | 28,153 | 0,00010 |

**Reaktionsbedingungen**

| T [°C] | P [bar] | Verhältnis n(H₂)/n(Dien) | Isomerisierung 1-Buten [%] |
|---|---|---|---|
| 96 | 24 | 2,06 | 44,87 |

### Beispiel 3 (gemäß Erfindung)

| Komponente | 1,3-Butadien | 1-Buten | 2-Butene | n-Butan | Ethanthiol |
|---|---|---|---|---|---|
| Zulauf [Gew.-%] | 0,502 | 29,489 | 46,949 | 22,502 | 0,00210 |
| Austrag [Gew.-%] | 0,325 | 29,276 | 47,381 | 22,461 | 0,00000 |

**Reaktionsbedingungen**

| T [°C] | P [bar] | Verhältnis n(H₂)/n(Dien) | Isomerisierung 1-Buten [%] |
|---|---|---|---|
| 122 | 24 | 0,30 | 0,75 |

### Beispiel 4 (gemäß Erfindung)

| Komponente | 1,3-Butadien | 1-Buten | 2-Butene | n-Butan | Ethanthiol |
|---|---|---|---|---|---|
| Zulauf [Gew.-%] | 0,185 | 26,140 | 45,398 | 27,808 | 0,00200 |
| Austrag [Gew.-%] | 0,111 | 25,867 | 45,787 | 27,766 | 0,00000 |

**Reaktionsbedingungen**

| T [°C] | P [bar] | Verhältnis n(H₂)/n(Dien) | Isomerisierung 1-Buten [%] |
|---|---|---|---|
| 118 | 24 | 0,54 | 1,04 |

### Beispiel 5 (gemäß Erfindung)

| Komponente | 1,3-Butadien | 1-Buten | 2-Butene | n-Butan | Methanthiol |
|---|---|---|---|---|---|
| Zulauf [Gew.-%] | 0,509 | 31,570 | 43,547 | 23,874 | 0,00207 |
| Austrag [Gew.-%] | 0,386 | 31,349 | 43,899 | 23,868 | 0,00000 |

**Reaktionsbedingungen**

| T [°C] | P [bar] | Verhältnis n(H₂)/n(Dien) | Isomerisierung 1-Buten [%] |
|---|---|---|---|
| 105 | 24 | 0,29 | 0,70 |

### Beispiel 6 (Vergleichsbeispiel)

| Komponente | 1,3-Butadien | 1-Buten | 2-Butene | n-Butan | Ethanthiol |
|---|---|---|---|---|---|
| Zulauf [Gew.-%] | 0,506 | 38,882 | 39,693 | 20,414 | 0,00200 |
| Austrag [Gew.-%] | 0,508 | 38,897 | 39,700 | 20,392 | 0,00199 |

**Reaktionsbedingungen**

| T [°C] | P [bar] | Verhältnis n(H₂)/n(Dien) | Isomerisierung 1-Buten [%] |
|---|---|---|---|
| 88 | 20 | 0,00 | -0,04 |

Die Beispieltabellen zeigen jeweils die wesentlichen Zusammensetzung des Zulauf- und des Ausgangsstromes des Festbettreaktors unter verschiedenen Reaktionsbedingungen (ohne Verunreinigungen).

In Beispiel 1 sind die Ergebnisse der Thioveretherung von ca. 5000 ppm 1,3-Butadien und ca. 21 ppm Ethanthiol bei einer erfindungsgemäßen Wasserstoffmenge gezeigt. Es ist zu sehen, dass Ethanthiol auf einen Massenanteil von 0 ppm verethert werden kann, ohne dass große Anteile am Wertprodukt 1-Buten verloren gehen. 1-Buten wird nur zu 0,59 % umgesetzt (Umsatz = (m_{Ein} - m_{Aus})/m_{Ein}).

In Beispiel 2 wird ein zu US5851383 analoger Wasserstoffüberschuss gegenüber 1,3-Butadien von 2 (mol/mol) eingestellt. Auch hier sind ca. 5000 ppm 1,3-Butadien und ca. 20 ppm Ethanthiol im Zulauf vorhanden. Bei dieser hohen Wasserstoffmenge wird der Ethanthiol-Massenanteil jedoch nur auf einen Wert von ca. 1,0 ppm reduziert, was bei der Feinreinigung von C₄-Schnitten nicht akzeptabel ist. Zusätzlich sinkt der 1-Butengehalt um über 44%, während der n-Butananteil als Zeichen für eine Totalhydrierung um 5000 ppm ansteigt.

In Beispiel 3 wird die Temperatur auf 122°C angehoben. Auch hier können bei ca. 5000 ppm 1,3-Butadien ca. 21 ppm Ethanthiol auf einen Massenanteil von 0 ppm thioverethert werden, ohne dass große Anteile an den Wertprodukten verloren gehen. 1-Buten wird zu nur 0,75% zu 2-Butenen umgesetzt, während der Anteil an Butanen als Zeichen für eine Totalhydrierung nicht ansteigt.

In Beispiel 4 wird die Zulaufkonzentration an 1,3-Butadien auf ca. 1000 ppm abgesenkt und gleichzeitig wird das Verhältnis Wasserstoff zu Dien von 0,30 auf 0,54 angehoben. Auch hier können ca. 20 ppm Ethanthiol auf einen Massenanteil von 0 ppm thioverethert werden, ohne dass große Anteile an den Wertprodukten verloren gehen. Durch das erhöhte Wasserstoff/Dien-Verhältnis werden jetzt 1,04% 1-Buten umgesetzt, was jedoch noch immer ein sehr kleiner Wert ist. Eine Totalhydrierung zu Butanen findet jedoch nicht statt.

In Beispiel 5 sind die Ergebnisse der Thioveretherung von ca. 5000 ppm 1,3-Butadien und ca. 21 ppm Methanthiol bei einer erfindungsgemäßen Wasserstoffmenge gezeigt. Es ist zu sehen, dass auch Methanthiol auf einen Massenanteil von 0 ppm verethert werden kann, ohne dass große Anteile am Wertprodukt 1-Buten verloren gehen. 1-Buten wird nur zu 0,70 % umgesetzt.

In Beispiel 6 wird ohne Wasserstoff im C₄-Kohlenwasserstoffstrom gearbeitet. Auch hier sind ca. 5000 ppm 1,3-Butadien und ca. 20 ppm Ethanthiol im Zulauf vorhanden. Ohne Wasserstoff wird der Ethanthiol-Massenanteil nicht beeinflusst, das heißt, eine Thioveretherung findet ohne Wasserstoffzufuhr nicht statt.

Abschließend sollen die Grundgedanken der Erfindung und ihr wesentlicher Nutzen noch einmal zusammengefasst werden:
Mit Mercaptanen verunreinigte C₄-Kohlenwasserstoffströme, welche meist aus katalytischen Crackern stammen, sind ungeeignet, einer Oligomerisierung unterzogen zu werden, da die Mercaptane den Oligomerisierungskatalysator stark schädigen. Bisher wurden derartige Ströme in einem kostenintesiven MEROX®-Prozess von Mercaptanen gereinigt, wobei jedoch die Entfernung der Mercaptane nicht vollständig gelang. Dies erforderte eine adsorbtive Entfernung der verbliebenen Mercaptane vor der Oligomerisierung. Der Erfindung liegt die Idee zu Grunde, alle im C₄-Strom vorhandenen Mercaptane zu einem höheren Molekulargewicht zu verethern, um eine destillative Abtrennung der entstandenen Thioether zu ermöglichen. Überraschend wurde gefunden, dass die Thioveretherung an einem heterogenen Katalysator erfolgen kann, wenn eine sehr geringe Menge an Wasserstoff zugegen ist. Ein großer Vorteil dieses Verfahrens ist, das aufgrund des geringen Wasserstoffanteils im C₄-Strom enthaltendes 1-Buten kaum isomerisiert wird und weiterhin als Wertprodukt zur Verfügung steht, sowie keine Vollhydrierung zu Butanen stattfindet. Darüber hinaus macht das Verfahren eine kostenintensive MEROX®-Wäsche verzichtbar.

## Patentansprüche

1. Verfahren zur Thioveretherung von Mercaptanen mit mehrfach ungesättigten Kohlenwasserstoffen, durchgeführt in einem Reaktor unter Zusatz von Wasserstoff, unter Einsatz eines heterogenen Katalysators und in Gegenwart von 1-Buten,
**dadurch gekennzeichnet,**
**dass** das molare Verhältnis von Wasserstoff zu mehrfach ungesättigten Kohlenwasserstoffen zwischen 0,01 und 0,8 beträgt.

2. Verfahren nach Anspruch 1, **dadurch gekennzeichnet, dass** das molare Verhältnis von Wasserstoff zu mehrfach ungesättigten Kohlenwasserstoffen zwischen 0,1 und 0,5 beträgt.

3. Verfahren nach Anspruch 1 oder 2, **dadurch gekennzeichnet, dass** die Mercaptane mit mehrfach ungesättigten Kohlenwasserstoffen thioverethert werden, welche ausgewählt sind aus der Gruppe umfassend:
1,3-Butadien, But-3-en-1-in und 1,2-Butadien.

4. Verfahren nach Anspruch 1, 2 oder 3, **dadurch gekennzeichnet, dass** Ethanthiol und/oder Methanthiol mit mehrfach ungesättigten Kohlenwasserstoffen thioverethert wird.

5. Verfahren nach einem der vorhergehenden Ansprüche, **dadurch gekennzeichnet, dass** es in Gegenwart von Kohlenmonoxid durchgeführt wird, wobei der Gehalt an Kohlenmonoxid im Zulauf des Reaktors weniger als 20 ppm beträgt, bezogen auf die Masse des Zulaufes.

6. Verfahren nach einem der vorhergehenden Ansprüche,
**dadurch gekennzeichnet,**
**dass** der heterogene Katalysator ein Metall der VIII. Gruppe des Periodensystems der Elemente enthält.

7. Verfahren nach Anspruch 6,
**dadurch gekennzeichnet,**
**dass** es sich bei dem Katalysator um einen Schalenkatalysator handelt, umfassend Aluminiumoxid als Träger und Palladium als katalytisch wirksames Metall.

8. Verfahren nach einem der vorhergehenden Ansprüche,
**dadurch gekennzeichnet,**
**dass** die Eintrittstemperatur des Zulaufs in den Reaktor zwischen 0°C und 180°C beträgt; vorzugsweise zwischen 60°C und 150°C und ganz besonders bevorzugt zwischen 80°C und 130°C.

9. Verfahren nach einem der vorhergehenden Ansprüche,
**dadurch gekennzeichnet,**
**dass** es als Flüssigphasenverfahren betrieben wird, dergestalt, dass der Wasserstoff in der flüssigen Phase vollständig gelöst ist.

10. Verfahren nach einem der Ansprüche 1 bis 9,
**dadurch gekennzeichnet,**
**dass** als Zulauf für den Reaktor ein Gemisch aus C₄-Kohlenwasserstoffen verwendet wird.

11. Verfahren nach Anspruch 10,
**dadurch gekennzeichnet,**
**dass** das als Zulauf verwendete Gemisch zuvor einer destillativen Abtrennung von Leichtsiedern, insbesondere von Isobutan unterzogen wird.

12. Verfahren nach mindestens einem der Ansprüche 1 bis 11,
**dadurch gekennzeichnet,**
**dass** auf die Thioveretherung mindestens ein Verfahrensschritt folgt, auswählt aus der Gruppe umfassend:
• Destillative Abscheidung von Thioethern;
• Adsorptive Entfernung von Schwefelverbindungen;
• Selektivhydrierung von 1,3-Butadien zu 1-Buten und/oder zu 2-Buten;
• Destillative Abtrennung von 1-Buten;
• Oligomerisierung von 2-Butenen zu Olefinen mit mehr als 4 Kohlenstoffatomen;
• Destillative Abtrennung von n-Butan und/oder von Isobutan;
• Veretherung von Isobuten mit Methanol zu Methyl-tert.-Butylether (MTBE) und Abtrennung des entstandenen MTBE.

13. Verfahren nach Anspruch 12, **dadurch gekennzeichnet, dass** auf die Thioveretherung eine kombinierte destillative Abtrennung von Thioethern und von 1-Buten erfolgt.

14. Verfahren nach Anspruch 12, wobei auf die Thioveretherung eine Veretherung von Isobuten mit Methanol zu Methyl-tert.-Butylether (MTBE) folgt, **dadurch gekennzeichnet, dass** danach eine destillative Abtrennung eines MTBE und Thioether umfassenden Ethergemisches erfolgt.

## Claims

1. Process for the thioetherification of mercaptans with multiply unsaturated hydrocarbons, carried out in a reactor with addition of hydrogen using a heterogeneous catalyst and in the presence of 1-butene,
**characterized in that**
the molar ratio of hydrogen to multiply unsaturated hydrocarbons is in the range from 0.01 to 0.8.

2. Process according to Claim 1, **characterized in that** the molar ratio of hydrogen to multiply unsaturated hydrocarbons is in the range from 0.1 to 0.5.

3. Process according to Claim 1 or 2, **characterized in that** the mercaptans are thioetherified with multiply unsaturated hydrocarbons which are selected from the group consisting of: 1,3-butadiene, but-3-en-1-yne and 1,2-butadiene.

4. Process according to Claim 1, 2 or 3, **characterized in that** ethanethiol and/or methanethiol is/are thioetherified with multiply unsaturated hydrocarbons.

5. Process according to any of the preceding claims, **characterized in that** it is carried out in the presence of carbon monoxide, with the content of carbon monoxide in the feed to the reactor being less than 20 ppm, based on the mass of the feed.

6. Process according to any of the preceding claims,
**characterized in that**
the heterogeneous catalyst contains a metal of group VIII of the Periodic Table of the Elements.

7. Process according to Claim 6,
**characterized in that**
the catalyst is a coated catalyst comprising aluminium oxide as support and palladium as catalytically active metal.

8. Process according to any of the preceding claims,
**characterized in that**
the entry temperature of the feed into the reactor is in the range from 0°C to 180°C; preferably from 60°C to 150°C and very particularly preferably from 80°C to 130°C.

9. Process according to any of the preceding claims,
**characterized in that**
it is operated as a liquid-phase process in such a way that the hydrogen is completely dissolved in the liquid phase.

10. Process according to any of Claims 1 to 9,
**characterized in that**
a mixture of C₄-hydrocarbons is used as feed for the reactor.

11. Process according to Claim 10,
**characterized in that**
the mixture used as feed is subjected beforehand to a removal of low boilers, in particular isobutane, by distillation.

12. Process according to at least one of Claims 1 to 11,
**characterized in that**
the thioetherification is followed by at least one process step selected from the group consisting of:
• removal of thioethers by distillation;
• removal of sulphur components by adsorption;
• selective hydrogenation of 1,3-butadiene to 1-butene and/or 2-butene;
• removal of 1-butene by distillation;
• oligomerization of 2-butenes to form olefins having more than 4 carbon atoms;
• removal of n-butane and/or of isobutane by distillation;
• etherification of isobutene with methanol to form methyl tert-butyl ether (MTBE) and removal of the MTBE formed.

13. Process according to Claim 12, **characterized in that** the thioetherification is followed by a combined removal of thioethers and of 1-butene by distillation.

14. Process according to Claim 12, wherein the thioetherification is followed by an etherification of isobutene with methanol to form methyl tert-butyl ether (MTBE), **characterized in that** after this a removal of an ether mixture comprising MTBE and thioether by distillation is carried out.

## Revendications

1. Procédé de thioéthérification de mercaptans avec des hydrocarbures polyinsaturés, réalisé dans un réacteur avec ajout d'hydrogène, en utilisant un catalyseur hétérogène et en présence de 1-butène,
**caractérisé en ce que** le rapport molaire entre l'hydrogène et les hydrocarbures polyinsaturés est compris entre 0,01 et 0,8.

2. Procédé selon la revendication 1, **caractérisé en ce que** le rapport molaire entre l'hydrogène et les hydrocarbures polyinsaturés est compris entre 0,1 et 0,5.

3. Procédé selon la revendication 1 ou 2, **caractérisé en ce que** les mercaptans sont thioéthérés avec des hydrocarbures polyinsaturés, qui sont choisis dans le groupe comprenant :
le 1,3-butadiène, le but-3-én-1-yne et le 1,2-butadiène.

4. Procédé selon la revendication 1, 2 ou 3, **caractérisé en ce que** de l'éthane-thiol et/ou du méthane-thiol sont thioéthérés avec des hydrocarbures polyinsaturés.

5. Procédé selon l'une quelconque des revendications précédentes, **caractérisé en ce qu'**il est réalisé en présence de monoxyde de carbone, la teneur en monoxyde de carbone dans l'alimentation du réacteur étant inférieure à 20 ppm, par rapport à la masse de l'alimentation.

6. Procédé selon l'une quelconque des revendications précédentes, **caractérisé en ce que** le catalyseur hétérogène contient un métal du groupe VIII du tableau périodique des éléments.

7. Procédé selon la revendication 6, **caractérisé en ce que** le catalyseur est un catalyseur à enveloppe comprenant de l'oxyde d'aluminium en tant que support et du palladium en tant que métal catalytiquement actif.

8. Procédé selon l'une quelconque des revendications précédentes, **caractérisé en ce que** la température d'entrée de l'alimentation dans le réacteur est comprise entre 0 °C et 180 °C, de préférence entre 60 °C et 150 °C et de manière tout particulièrement préférée entre 80 °C et 130 °C.

9. Procédé selon l'une quelconque des revendications précédentes, **caractérisé en ce qu'**il est réalisé sous la forme d'un procédé en phase liquide, de sorte que l'hydrogène soit entièrement dissous dans la phase liquide.

10. Procédé selon l'une quelconque des revendications 1 à 9, **caractérisé en ce qu'**un mélange d'hydrocarbures en C₄ est utilisé en tant qu'alimentation pour le réacteur.

11. Procédé selon la revendication 10, **caractérisé en ce que** le mélange utilisé en tant qu'alimentation est auparavant soumis à une séparation par distillation des composants de point d'ébullition faible, notamment de l'isobutane.

12. Procédé selon au moins l'une quelconque des revendications 1 à 11, **caractérisé en ce que** la thioéthérification est suivie par au moins une étape de procédé, choisie dans le groupe comprenant :
- une séparation par distillation de thioéthers ;
- une élimination par adsorption de composés soufrés ;
- une hydrogénation sélective de 1,3-butadiène en 1-butène et/ou en 2-butène ;
- une séparation par distillation de 1-butène ;
- une oligomérisation de 2-butènes en oléfines contenant plus de 4 atomes de carbone ;
- une séparation par distillation de n-butane et/ou d'isobutane ;
- une éthérification d'isobutène avec du méthanol en éther de méthyle et de tert.-butyle (MTBE) et une séparation du MTBE formé.

13. Procédé selon la revendication 12, **caractérisé en ce que** la thioéthérification est suivie par une séparation par distillation combinée de thioéthers et de 1-butène.

14. Procédé selon la revendication 12, dans lequel la thioéthérification est suivie par une éthérification d'isobutène avec du méthanol en éther de méthyle et de tert.-butyle (MTBE), **caractérisé en ce qu'**une séparation par distillation d'un mélange d'éthers comprenant du MTBE et des thioéthers a ensuite lieu.
